# EUROPEAN PATENT SPECIFICATION

(11) **EP 4 757 934 B1**
(45) Date of publication and mention of the grant of the patent: **11.11.2026**
(21) Application number: 25796537.6
(22) Date of filing: 29.10.2025
(51) Int. Cl.: B01J 23/30, C07D 201/00, B01J 38/48, B01J 31/40

(54) **METHOD FOR REACTIVATING A HOMOGENEOUS CATALYST SYSTEM FOR AN OXIDATION REACTION**
VERFAHREN ZUR REAKTIVIERUNG EINES HOMOGENEN KATALYSATORSYSTEMS FÜR EINE OXIDATIONSREAKTION
PROCÉDÉ DE RÉACTIVATION D'UN SYSTÈME CATALYSEUR HOMOGÈNE POUR UNE RÉACTION D'OXYDATION

(30) Priority: 30.10.2024 EP 24209710
(43) Date of publication of application: 17.06.2026
(73) Proprietor: Evonik Operations GmbH, 45128 Essen (DE)
(72) Inventor: VOGELSANG, Dennis, 48249 Dülmen (DE); LIENKE, Achim, 3124ND Schiedam (NL)
(74) Representative: Evonik Patent Association
(86) International application number: PCT/EP2025/081235
(87) International publication number: WO 2026/093374

(56) References cited:
- EP-A2- 2 946 831
- WO-A1-2018/002114

## Description

The present invention relates to a process for reactivating a homogeneous catalyst system for the oxidation of an ethylenically unsaturated organic compound. Moreover, the invention relates to methods for the oxidation of an ethylenically unsaturated organic compound and for synthesizing a lactam, and biphasic mixtures for use in or obtained in such reactions, respectively.

Metal peroxo-complexes and in particular polyoxometalates play a crucial role in the homogeneous catalysis of various oxidation reactions of organic compounds. For instance, C. Venturello et al. describe a process for the epoxidation of olefins, such as 1-octene and cyclohexene, using hydrogen peroxide in the presence of a polytungstophosphate catalyst and a tetraalkylammonium phase transfer catalyst, such as methyltrioctylammonium chloride, in a biphasic solvent mixture (J. Org. Chem. 1983, 48, 3831-3833). Technical applications include the usage of such complexes for the epoxidation of cyclododecene (CDEN) with hydrogen peroxide as oxidant in a biphasic reaction mixture as described in WO 2018/002114 A1, EP 2 946 831 A2 and WO 2021/085978A1, or in the synthesis of 1,2-propanediol as described in WO 2023/152083 A1. In such oxidation reactions carried out in biphasic systems, a phase transfer catalyst, such as a ternary or quaternary ammonium compound or an ester quat, is typically employed.

However, it has been observed that in continuous processes the conversion rate of an oxidation reaction at a constant catalyst concentration decreases over time and thus, the addition of fresh catalyst system becomes necessary. As a result, the catalyst system progressively concentrates over time when a constant conversion rate is maintained. The active catalyst seems to be converted into a less reactive species adversely affecting the reaction rate. With increasing amounts of the catalyst system being added, the catalyst system can precipitate in parts of the reactor, finally clogging parts of the reactor or blocking membranes, requiring a discontinuation of the process for maintenance.

Moreover, the addition of fresh catalyst system leads to increased costs and environmental impact. Thus, a general problem in the technical application of such homogeneous catalysts is the recycling of the catalyst system.

In EP 2 946 831 A2 a method for separating a homogeneous catalyst system from an organic phase of a reaction mixture using a membrane comprising a membrane separation-active layer is disclosed.

WO 2018/002114 A1 describes a process for reactivating homogeneous catalyst systems from organic reaction mixtures, wherein after completion of the oxidation reaction the catalyst systems are first treated with an aqueous base, followed by acidification of the resulting mixture comprising the catalyst systems to a pH of at most 4 before the mixture can be recycled into the same oxidation reaction.

However, there still remains a need for an improved method for the reactivation of a homogeneous catalyst system.

It has now surprisingly been found that when using a permanently ionized phase transfer reagent the decrease in conversion rate of a continuous oxidation process can be minimized if the catalyst system is reactivated and recycled into the reactor in form of a biphasic mixture having a basic pH. This is particularly surprising since a peroxide can easily decompose in an alkaline medium and therefore contact of the peroxide with a basic reaction mixture is normally avoided. By using the present method, the need for adding fresh catalyst system in continuous processes and the associated problems can be minimized or even prevented.

Accordingly, the present invention relates to a method for reactivating a homogeneous catalyst system for the oxidation of an ethylenically unsaturated organic compound with a peroxide as oxidant in a reaction mixture comprising an organic phase, an acidic aqueous medium and a permanently ionized phase transfer reagent, comprising the steps of
(i) separating the organic phase comprising at least a part of the catalyst system from the aqueous medium;
(ii) concentrating the catalyst system in the organic phase;
(iii) adding at least one aqueous base to the organic phase comprising the catalyst system obtained in step (ii) to form a biphasic mixture having a pH of at least 7.0, determined in the stirred biphasic mixture using a pH electrode; and
(iva) feeding the biphasic mixture obtained in step (iii) to a reaction mixture comprising an ethylenically unsaturated organic compound, a peroxide, and an acidic aqueous medium without separating the aqueous phase from the organic phase and without acidifying said mixture before feeding it to the reaction mixture; or
(ivb) treating either the biphasic mixture obtained in step (iii) or, after phase separation, the aqueous phase thereof, with an acid in the presence of a peroxide before feeding the treated biphasic mixture or both, the treated aqueous phase and the organic phase obtained from separating the biphasic mixture, respectively, to a reaction mixture comprising an ethylenically unsaturated organic compound, and an acidic aqueous medium;
wherein the catalyst system comprises at least one derivative of a transition metal of Group IVb, Vb and Vlb in its highest oxidation state.

Moreover, the present invention relates to a method for the oxidation of an ethylenically unsaturated organic compound, comprising the steps of
(i) oxidizing an ethylenically unsaturated organic compound with a peroxide in a reaction mixture comprising a homogeneous catalyst system, an organic phase, an acidic aqueous medium and a permanently ionized phase transfer reagent; wherein the homogeneous catalyst system comprises at least one derivative of a transition metal of Group IVb, Vb and Vlb in its highest oxidation state; and
(ii) reactivating the catalyst system of the reaction mixture according to the method described herein.

Furthermore, the present invention relates to a method for synthesizing a lactam, comprising: epoxidizing a cyclic ethylenically unsaturated compound to an epoxide; rearranging the epoxide to a ketone; converting the ketone to an oxime; and rearranging the oxime to give the lactam;
wherein the cyclic unsaturated compound is epoxidized by at least one peroxide in a reaction mixture comprising an organic phase, an acidic aqueous medium, a permanently ionized phase transfer reagent and a catalyst system comprising at least one derivative of a transition metal of Group IVb, Vb and Vlb in its highest oxidation state, and wherein the catalyst system is reactivated according to the method described herein.

In addition, the present invention relates to a biphasic mixture for use in an oxidation of an ethylenically unsaturated organic compound, comprising an oxidation product of the ethylenically unsaturated organic compound, an aqueous phase and a catalyst system as described herein, wherein the biphasic mixture has a pH value of at least 7.0, determined in the stirred biphasic mixture using a pH electrode, preferably of from 7.0 to 11.0, more preferably of from 8.0 to 10.0, and most preferably from 8.0 to 9.0.

Moreover, the present invention relates to a biphasic mixture obtained from an epoxidation process comprising an organic phase and an aqueous phase, wherein the organic phase comprises an epoxide in an amount of at least 50 wt.-%, preferably of at least 70 wt.-%, more preferably of at least 80 wt.-%, a transition metal of the Group IVb, Vb and Vlb in an amount of less than 6000 ppm, preferably less than 1500 ppm, and a quaternary ammonium compound in an amount of more than 0.2 wt.-%, preferably more than 0.5 wt.-%, more preferably more than 1.0 wt.-%, wherein ppm and wt.-% are based on the total amount of the organic phase, and wherein the aqueous phase comprises the same transition metal as the organic phase in such an amount that more than 50 wt.-% of the total amount of said transition metal in the biphasic mixture is in the aqueous phase, preferably more than 75 wt.-%, more preferably more than 85 wt.-%, and wherein the biphasic mixture has a pH of at least 7.0, determined in the stirred biphasic mixture using a pH electrode, preferably of from 7.0 to 11.0, more preferably of from 8.0 to 10.0, most preferably of from 8.0 to 9.0.

As used herein, the term "comprising" and variations thereof are used synonymously with the terms "including", "containing" and variations thereof and are understood to be open and non-limiting terms which do not exclude the presence of additional undescribed or unrecited elements, compounds, ingredients or process steps. As used herein, the term "consisting of" is understood to exclude the presence of unspecified elements, compounds, ingredients or process steps. When the non-limiting terms "comprising", "including" or "containing" are used in the present specification, the case of "consisting of" is included therein. For example, a process described as "comprising" or "including" certain steps can consist of the explicitly recited steps or can further comprise one or more unrecited steps. Same applies, for example, to compositions and their corresponding explicitly described or non-recited ingredients.

In this application, the indefinite article "a" means one or more of what it denotes.

As stated above, the present invention relates to a method for reactivating a homogeneous catalyst system for the oxidation of an ethylenically unsaturated organic compound with a peroxide as oxidant in a reaction mixture comprising an organic phase, an acidic aqueous medium and a permanently ionized phase transfer reagent.

According to the present invention, the oxidation of an ethylenically unsaturated organic compound can be an epoxidation of an ethylenically unsaturated organic compound. In the oxidation according to the present invention a peroxide is used as an oxidant. Suitable peroxides are known to those skilled in the art and include 3-chloroperoxybenzoic acid, peroxybenzoic acid, peroxyacetic acid, peroxybenzimidic acid, tert-butylhydroperoxide, dimethyldioxirane, potassium hydrogen peroxomonosulfate and hydrogen peroxide, wherein hydrogen peroxide is the preferred peroxide.

As used herein, the term "ethylenically unsaturated organic compound" refers to an organic compound having at least one carbon-carbon double bond and/or at least one carbon-carbon triple bond, preferably at least on carbon-carbon double bond. Preference is given to ethylenically unsaturated organic compounds having a solubility in water at 20 °C of no more than 1 wt.-%, preferably of no more than 0.5 wt.-%. A solubility in water at 20 °C of 1 wt.-%, as used herein, means that at a temperature of 20 °C no more than 10 g of the respective compound dissolves in 1000 g of distilled water. The ethylenically unsaturated organic compound preferably may be an organic compound having a total of six to twenty carbon atoms, wherein such compounds more preferably may be cyclic compounds. Particularly preferred are cyclic unsaturated C12 compounds, especially cyclododecene (CDEN).

As used herein, the term "oxidized ethylenically unsaturated organic compound" refers to any product resulting from oxidizing at least one carbon-carbon double of the ethylenically unsaturated organic compound, such as, but not limited to the corresponding epoxide.

The reaction mixture of the oxidation comprises an organic phase, an acidic aqueous medium and a permanently ionized phase transfer reagent.

As used herein, the term "acidic aqueous medium" refers to a liquid phase comprising at least 50 wt.-% water, based on the total weight of the liquid phase, and having a pH of less than 7.0, preferably a pH of 4.0 or less, more preferably a pH of 1.5 to 4.0 and most preferably a pH of 1.5 to 2.5. The pH is preferably adjusted with at least one inorganic acid having a pKₐ of 2.5 or less at 25 °C. Suitable acids are selected from phosphoric acid, nitric acid, sulfuric acid, hydrochloric acid, perchloric acid and mixtures thereof, preferably from phosphoric acid and sulfuric acid or mixtures thereof. Most preferably the acid is sulfuric acid.

As used herein the term "pH" or "pH value" refers to the "apparent pH", which refers to a value determined by measurement with a glass electrode employing a commercial pH meter calibrated with aqueous buffer solutions of known pH for measuring dilute aqueous solutions. This apparent pH differs from the notional pH, i.e., the negative logarithm of the hydrogen ion activity, by a constant value because the normal potential of the glass electrode in the aqueous phase of the reaction mixture, which comprises hydrogen peroxide and alcohols, is different than the normal potential in pure water.

The oxidation may be carried out in an organic solvent or can be carried out in the absence of an additional solvent, if the ethylenically unsaturated organic compound itself functions as solvent.

The "organic phase" of the reaction mixture can comprise the ethylenically unsaturated organic compound, the oxidized ethylenically unsaturated organic compound, at least a part of the catalyst system, at least a part of the permanently ionized phase transfer reagent and, in case an organic solvent is used, the organic solvent.

As used herein the term "permanently ionized phase transfer reagent" refers to a phase transfer reagent comprising a compound having a charge independent of the pH. For instance, in contrast to primary, secondary or tertiary alkylamino groups which are reversibly protonated and thus ionized dependent upon the pH, quaternary alkyl ammonium groups have a positive charge independent of the pH. A phase transfer reagent as used herein refers to a compound that facilitates the transition of another compound, e.g., a catalyst or a reactant, from one phase of a reaction mixture into another phase in which a reaction occurs. The permanently ionized phase transfer reagent according to the present invention preferably may comprise a quaternary ammonium compound.

The quaternary ammonium compound preferably comprises an ammonium cation of the general formula N⁺R¹R²R³R⁴, wherein R¹, R², R³, and R⁴ are alkyl groups which each independently may be the same or different. Herein the ammonium cation has in total more than 10, preferably more than 15 carbon atoms in the alkyl groups. More preferably at least one of R', R², R³, and R⁴ is a methyl group. Special preference is given to permanently ionized phase transfer reagents comprising trialkylammonium methyl salts, wherein the alkyl chains each consist of six to twelve carbon atoms, such as methyltrioctylammonium sulfate or Adogen^{®} 464, which is a tri-(C₈-C₁₀)-alkylammonium methyl salt available, for instance, from Merck KGaA (Darmstadt, Germany).

The homogeneous catalyst system reactivated in the method according to the present invention comprises at least one derivative of a transition metal of Group IVb, Vb and Vlb in its highest oxidation state.

As used herein, the term "homogeneous catalyst system" is to be understood in the way it is generally understood by a skilled person in the field of organic synthesis, namely as a catalyst system used in homogeneous catalysis, wherein the catalyst is in the same phase as the reactants such as in case of a soluble catalyst in a solution. The homogeneous catalyst system catalyzes the oxidation of the ethylenically unsaturated organic compound in the biphasic reaction mixture.

As used herein, the term "transition metal of Group IVb" refers to an element that is in Group IVb of the CAS version of the Periodic Table of the Elements as is shown, for example, in the Handbook of Chemistry and Physics, 63rd edition (1983), corresponding to Group 4 in the actual IUPAC numbering. Likewise, the terms "transition metal of Group Vb" and "transition metal of Group Vlb" refer to an element that is in Group Vb and Vlb, respectively, of the CAS version of the Periodic Table of the Elements as is shown, for example, in the Handbook of Chemistry and Physics, 63rd edition (1983), corresponding to Group 5 and 6, respectively, in the actual IUPAC numbering.

The at least one derivative of the transition metal of Group IVb, Vb and Vlb preferably may be selected from a derivative of tungsten (oxidation state 6), molybdenum (oxidation state 6) and vanadium (oxidation state 5).

Suitable derivatives include, for example, oxides, mixed oxides, oxygen-containing acids, salts of oxygen-containing acids, carbonyl derivatives, sulfides, chlorides, oxychlorides and alkanoates of tungsten, molybdenum and/or vanadium.

Preference is given to derivatives selected from salts of tungstic acid (H₂WO₄) or molybdic acid (H₂MoO₄), or homo- or heteropolyoxometalates formed therefrom. Special preference is given to derivatives selected from alkali or alkaline earth metal salts of H₂WO₄ or H₂MoO₄, or homo- or heteropolyoxometalates formed therefrom, in particular to Na₂WO₄ or a homo- or heteropolyoxometalate formed therefrom.

A polyoxometalate is a polyatomic ion, usually an anion, comprising three or more transition metal oxyanions linked together by shared oxygen atoms to form closed 3-dimensional frameworks. Homopolyoxometalates are composed of only one kind of metal and oxygen, while heteropolyoxometalates are composed of one or more metals, oxygen and eventually a main group oxyanion, such as a phosphate or a silicate.

These derivatives can be formed or converted into the catalytically active species *in situ.* For instance, the catalyst system can further comprise phosphoric acid, a salt of phosphoric acid or a combination of both. For example, sodium tungstate can be used in combination with phosphoric acid to form a catalytically active polyoxometalate *in situ.* Phosphoric acid or salts thereof can, for instance, be used as a stabilizer for hydrogen peroxide and thus, if hydrogen peroxide is used as the oxidant, be added to the reaction mixture in this form in combination with the hydrogen peroxide.

In the first step (step (i)) of the present method for reactivating the homogeneous catalyst system, the organic phase of the reaction mixture is separated from the aqueous phase of the reaction mixture, wherein at least a part of the catalyst system is retained the organic phase. Said separation can preferably be carried out by using a phase separation vessel.

The organic phase separated in step (i) may comprise residues of the ethylenically unsaturated organic compound, the oxidized ethylenically unsaturated organic compound, at least a part of the catalyst system, and at least a part of the permanently ionized phase transfer agent.

The aqueous phase from which the organic phase is separated in step (i) may comprise water, peroxide, a part of the catalyst system, and a part of the permanently ionized phase transfer agent.

In the second step (step (ii)) of the present method for reactivating the homogeneous catalyst system, the catalyst system is concentrated in the organic phase. Herein the oxidized ethylenically unsaturated organic compound and, if used, the solvent can be separated from the catalyst system. This results in the concentration of the catalyst system in the remaining organic phase. This can be effected for example by means of distillation, extraction, crystallization or membrane filtration, wherein membrane filtration is preferred. The step of concentrating the catalyst system in the organic phase preferably comprises separating the oxidized organic compound from the organic phase comprising the catalyst system by membrane filtration. Suitable membranes and process parameters for membrane filtration are described, for instance, in EP 2 946 831 A2. The membrane used for filtering the organic phase in step (ii) preferably comprises a membrane material based on silicone acrylate, polydimethylsiloxane (PDMS), polyimide or combinations thereof. Suitable commercially available membranes for concentrating the catalyst system in the organic phase are, for example, PuraMem^{®} S600 from Evonik Operations GmbH (Marl, Germany) and oNF-2^{®} from BORSIG Membrane Technology GmbH (Rheinfelden, Germany). The membrane can be a part of a continuous membrane system having multiple membrane modules.

When using membrane filtration for concentrating the catalyst system, the catalyst system is concentrated in the retentate. The term "retentate", as used herein, refers to the effluent from the membrane withdrawn upstream of the membrane. The material which passes through the membrane is known as "permeate" and is withdrawn downstream of the membrane. In the method according to the present invention, the permeate mainly comprises a mixture of the oxidized ethylenically unsaturated organic compound and the ethylenically unsaturated compound, i.e., the product of the oxidation reaction, meaning that the permeate comprises the mixture in an amount of at least 50 wt.-%, based on the total weight of the permeate. The amount of catalyst system retained in the remaining organic phase, i.e., the retentate, can be determined based on the amount of retained transition metal. In particular, the method allows retention of more than 50 wt.-% of the transition metal in the retentate, preferably more than 70 wt.-% and more preferably more than 90 wt.-%, based on the total amount of transition metal in the organic phase prior to filtration. The amount of retained transition metal may be detected using ICP-MS (inductively coupled plasma mass spectrometry) or XRF (X-ray fluorescence analysis).

As stated above, the organic phase can further comprise at least a part of the permanently ionized phase transfer reagent. If so, the permanently ionized phase transfer reagent is also concentrated in the retentate, if membrane filtration is used in step (ii) of the method according to the present invention.

Herein the permanently ionized phase transfer reagent can be retained at a different percentage than the transition metal.

In the third step (step (iii)) of the present method for reactivating the homogeneous catalyst system, at least one aqueous base is added to the organic phase comprising the catalyst system obtained in step (ii) to form a biphasic mixture having a pH of at least 7.0, determined in the stirred biphasic mixture using a pH electrode. The formed biphasic mixture preferably has a pH of from 7.0 to 11.0, more preferably of from 8.0 to 10.0 and most preferably of from 8.0 to 9.0.

The term "biphasic mixture" as used herein refers to a mixture comprising two liquid phases being immiscible at ambient temperature, namely an aqueous phase and an organic phase. Unless stated otherwise, "ambient temperature" or "room temperature" as used herein refers to a temperature of 23 °C.

The aqueous base added in step (iii) preferably may comprise ammonia, at least one alkali metal hydroxide or mixtures thereof, wherein the alkali metal hydroxide more preferably may be selected from sodium hydroxide and potassium hydroxide.

In a first variant of the fourth step (step (iva)) of the present method for reactivating the homogeneous catalyst system, the biphasic mixture obtained in step (iii) is fed to a reaction mixture comprising an ethylenically unsaturated organic compound, a peroxide and an acidic aqueous medium without separating the aqueous phase from the organic phase of the mixture obtained in step (iii) and without acidifying said mixture before feeding it to the reaction mixture. The latter means, that the biphasic mixture when fed to the reaction mixture still has a pH of at least 7.0, preferably of from 7.0 to 11.0, more preferably of from 8.0 to 10.0 and most preferably of from 8.0 to 9.0.

Alternatively, in a second variant of the fourth step (step (ivb)) of the present method for reactivating the homogeneous catalyst system, the either biphasic mixture obtained in step (iii) or, after phase separation, the aqueous phase thereof, is treated with an acid in the presence of a peroxide, preferably in the presence of hydrogen peroxide, before feeding the treated biphasic mixture or both, the treated aqueous phase and the organic phase obtained from separating the biphasic mixture, respectively, to a reaction mixture comprising an ethylenically unsaturated organic compound and an acidic aqueous medium. Since peroxides are prone to decomposition under alkaline conditions, it may be advantageous to add the peroxide shortly, e.g., within 5 minutes or less, after adding the acid to the biphasic medium or the aqueous phase thereof, respectively, to acidify it. In this way, the peroxide and the acid are present during the treatment at the same time and may react with the catalyst species while minimizing the amount of peroxide lost due to decomposition. Further, it may be advantageous to let react the mixture obtained from adding the acid and the peroxide to the biphasic mixture or the aqueous phase thereof, respectively, for some time, for instance for at least 5 minutes, or 15 minutes, or 30 minutes, or 45 minutes, or 1 hour and to optionally remove any solids formed or remaining therein during said time, e.g., by filtration, before feeding the treated biphasic mixture or both, the treated aqueous phase and the organic phase obtained from separating the biphasic mixture, respectively, to the reaction mixture comprising an ethylenically unsaturated organic compound and an acidic aqueous medium. It has been observed that removing any solids formed or remaining during the treatment of the biphasic mixture or the aqueous phase thereof, respectively, with an acid and hydrogen peroxide may even further enhance the activity of the reactivated catalyst. Without wishing to being bound by theory, it is believed that an inhibiting species is formed in the preceding oxidation reaction and/or any of the steps for reactivating the catalyst, the removal of which further enhances the activity of the reactivated catalyst. If, however, a filtration step is carried out before the biphasic mixture obtained in step (iii) or the aqueous phase thereof, respectively, has been treated with an acid in the presence of a peroxide, and the peroxide is added to said acidic mixture only after the filtration, a drop in the catalyst's activity is observed.

The reaction mixture to which the biphasic mixture or both, the treated aqueous phase and the organic phase obtained from separating the biphasic mixture, respectively, is fed in steps (iva) or (ivb) may further comprise a permanently ionized phase transfer reagent. This is particularly the case if the organic phase separated and concentrated in steps (i) and (ii) does not contain the entire amount of permanently ionized phase transfer reagent of the initial reaction mixture, so as to account for the partial loss in phase transfer reagent upon reactivating the catalyst system according to the method of the present invention.

In the method according to the present invention the pH of the biphasic mixture obtained in step (iii) is not adjusted to less than 7.0 before the biphasic mixture is contacted with a peroxide, such as hydrogen peroxide, in either step (iva) or (ivb).

After adding the at least one aqueous base and forming the biphasic mixture having a pH of at least 7.0 in step (iii) of the method, steps (iva) or (ivb) can either be performed immediately thereafter, i.e., in a continuous process or the resulting biphasic mixture obtained in step (iii) can be stored intermittently prior to carrying out step (iva) or (ivb) (batch process). Herein, preference is given to the continuous process.

By separating the organic phase in step (i), a part of the catalyst system can be removed with the aqueous phase. This portion of the catalyst system can optionally be recovered at least in part by further carrying out optional steps (v) and (vi).

Accordingly, the method according to the present invention may further comprise a step (v) of feeding the aqueous phase obtained by the separation in step (i) across at least one membrane and a step (vi) of feeding the retentate obtained in step (v) to a reaction mixture comprising an ethylenically unsaturated organic compound, a peroxide and an acidic aqueous medium.

The membrane used in step (v) for filtering the aqueous phase preferably may comprise a membrane material selected from the following: polyamides, aromatic polyamides, polysulphones, polyethersulphones, hydrophobized polyethersulphones, sulphonated polyethersulphones, cellulose acetate, polypiperazine and polyvinylidene fluoride. The membrane can be a part of a continuous membrane system having multiple membrane modules.

The aqueous phase in step (v) can further comprise a permanently ionized phase transfer reagent and the reaction mixture to which the retentate is then fed in step (vi) preferably is the same reaction mixture as described above for step (iva) or (ivb).

As an alternative to steps (v) and (vi), the aqueous phase obtained in step (i) of the method for reactivating a homogeneous catalyst system can be discarded.

The reaction mixture from which the catalyst system can be reactivated according to the method described herein preferably may be obtained in a continuous process which may be carried out in one stirred tank reactor or in a series of stirred tank reactors. This series of stirred tank reactors preferably comprises at least two stirred tank reactors, more preferably three to five stirred tank reactors.

The method for reactivating a homogeneous catalyst system according to the present invention allows to recover and reactivate a homogeneous catalyst system after its use in an oxidation reaction. This enables a very economical operation of the oxidation reaction. In particular, in a continuous process this method offers a great advantage as the amount of freshly added catalyst can be reduced to a minimum, thus reducing costs and environmental impact.

The present invention further relates to a method for the oxidation of an ethylenically unsaturated organic compound, in which a homogeneous catalyst system is used which is reactivated according to the method described herein above.

The method for the oxidation of an ethylenically unsaturated organic compound comprises the steps of (i) oxidizing an ethylenically unsaturated organic compound with a peroxide in a reaction mixture comprising a homogeneous catalyst system, an organic phase, an acidic aqueous medium and a permanently ionized phase transfer reagent, wherein the homogeneous catalyst system comprises at least one derivative of a transition metal of Group IVb, Vb and Vlb in its highest oxidation state, and (ii) reactivating the catalyst system of the reaction mixture according to the method described herein above.

The oxidation according to the method of the present invention is preferably carried out at a pH of 4.0 or less, more preferably at a pH of 1.5 to 4.0, even more preferably 1.5 to 2.5. Oxidation reactions employing polyoxometallates are pH dependent and the optimal pH window has to be determined experimentally. For example, Venturello et al. teaches that the reactivity increases with decreasing pH, but also the stability of the epoxide decreases with decreasing pH (J. Org. Chem. 1983, 48, 3831 -3833).

The ethylenically unsaturated organic compound oxidized in the method for oxidation according to the present invention refers to the ethylenically unsaturated organic compound as described above. Likewise, the peroxide, the organic phase, the acidic aqueous medium, the permanently ionized phase transfer reagent and the derivative of a transition metal of Group IVb, Vb and Vlb refer to the compounds as described above, respectively.

In a preferred embodiment of the present invention, the oxidation of the unsaturated organic compound is carried out in a continuous process in a cascade of stirred tank reactors. Herein, the ethylenically unsaturated organic compound, the homogeneous catalyst system in form of an aqueous solution and the permanently ionized phase transfer reagent are fed to a first stirred tank reactor of a cascade of stirred tank reactors to form a reaction mixture. The pH of the reaction mixture may preferably be adjusted to 4.0 or less by adding an acid. The conversion rate of the ethylenically unsaturated organic compound in the first reactor is at least 50%. The reaction mixture is then transferred to at least one further stirred tank reactor, before being fed to a phase separation vessel. Typical residence times in the first and second reactor independently are within the range of from 0.5 h to 24 h, such as 1 h to 12 h. In the phase separation vessel, the organic phase is separated from the aqueous phase and the aqueous phase may either be discarded or may be fed across at least one membrane and the obtained retentate may then be returned into the first stirred tank reactor. The organic phase may be fed across a membrane in which the oxidized organic compound is withdrawn downstream of the membrane as the permeate and the catalyst system is concentrated in the retentate, i.e., the remaining organic phase. The organic phase comprising the concentrated catalyst system is transferred into a further stirred tank reactor and an aqueous base is added to form a biphasic mixture having a pH of at least 7.0, determined in the stirred biphasic mixture using a pH electrode. The biphasic mixture is then returned into the first stirred tank reactor containing a reaction mixture comprising an ethylenically unsaturated organic compound, a peroxide and an acidic aqueous medium.

The present invention further relates to a method for synthesizing a lactam, comprising: epoxidizing a cyclic ethylenically unsaturated compound to an epoxide, rearranging the epoxide to a ketone, converting the ketone to an oxime, and rearranging the oxime to the lactam, wherein the cyclic ethylenically unsaturated compound is epoxidized by at least one peroxide in a reaction mixture comprising an organic phase, an acidic aqueous medium, a permanently ionized phase transfer reagent and a catalyst system comprising at least one derivative of a transition metal of Group IVb, Vb and Vlb in its highest oxidation state, and wherein the catalyst system is reactivated according to the method described herein above.

The method for synthesizing a lactam is particularly suitable for synthesizing laurolactam. The cyclic ethylenically unsaturated compound preferably may comprise 6 to 20 carbon atoms, more preferably 12 carbon atoms and most preferably is CDEN.

In the method for synthesizing a lactam according to the present invention, the catalyst system is reactivated according to the method described herein above after the step of epoxidizing a cyclic ethylenically unsaturated compound to an epoxide.

In the first step of this method, the organic compound is oxidized to the corresponding epoxide. The epoxidation is carried out in the presence of the homogeneous catalyst system reactivated by the method described herein above. The epoxidation is preferably carried out using the method for oxidation described herein above. The epoxidized compound is subsequently rearranged, for instance in the presence of a catalyst comprising a noble metal and a metal oxide, to the corresponding ketone. During said rearrangement or subsequent thereto, hydrogen can be added, so that the corresponding alcohol is formed. If the ketone is present in a mixture with the alcohol derivative, a dehydrogenation of the alcohol to the ketone can take place. The ketone is then converted to an oxime and finally the lactam is obtained by subsequent Beckmann rearrangement using e.g., sulfuric acid or cyanuric chloride. The rearrangement of the epoxide to the ketone and the subsequent steps are disclosed, for instance, in EP 2 772 478 A1. The resulting lactam can be subjected to further processing by polycondensation to give polyamides. A method for synthesizing laurolactam from CDEN via 1,2-epoxycyclododecane (CDAN epoxide) as an intermediate is particularly preferred. The obtained laurolactam can be polymerized to nylon 12 afterwards.

The present invention further relates to a biphasic mixture for use in an oxidation of an ethylenically unsaturated organic compound, comprising an oxidation product of the ethylenically unsaturated organic compound, an aqueous phase and a catalyst system as defined herein before, wherein the biphasic mixture has a pH value of at least 7.0, determined in the stirred biphasic mixture using a pH electrode, preferably of from 7.0 to 11.0, more preferably of from 8.0 to 10.0, most preferably of from 8.0 to 9.0. Said biphasic mixture may be obtained in step (iii) of the above-described method for reactivating a homogeneous catalyst system. The biphasic mixture comprises an organic phase and an aqueous phase. The oxidation product of the ethylenically unsaturated organic compound is comprised in the organic phase. The biphasic mixture can further comprise unreacted ethylenically unsaturated organic compound.

The biphasic mixture can be used for the oxidation of an ethylenically unsaturated organic compound according to the method described herein above, and is particularly suitable for use in a continuous process.

The present invention further relates to a biphasic mixture obtained from an epoxidation process comprising an organic phase and an aqueous phase, wherein the organic phase comprises an epoxide in an amount of at least 50 wt.-%, preferably of at least 70 wt.-%, more preferably of at least 80 wt.-%, a transition metal of the Group IVb, Vb and Vlb in an amount of less than 6000 ppm, preferably less than 1500 ppm and a quaternary ammonium compound in an amount of more than 0.2 wt.-%, preferably more than 0.5 wt.-%, more preferably more than 1.0 wt.-%, wherein ppm and wt.-% are based on the total amount of the organic phase, and wherein the aqueous phase comprises the same transition metal as the organic phase in such an amount that more than 50 wt.-% of the total amount of said transition metal in the biphasic mixture is in the aqueous phase, preferably more than 75 wt.-%, more preferably more than 85 wt.-%, and wherein the biphasic mixture has a pH of at least 7.0, determined in the stirred biphasic mixture using a pH electrode, preferably of from 7.0 to 11.0, more preferably of from 8.0 to 10.0, most preferably of from 8.0 to 9.0.

The transition metal in the biphasic mixture obtained from an epoxidation process preferably is tungsten, molybdenum or vanadium, more preferably tungsten. The amount of the transition metal in the biphasic mixture can be determined by X-ray fluorescence spectroscopy using, for instance, a SPECTRO XEPOS spectrometer of type 16004851 from SPECTRO Analytical Instruments GmbH (Kleve, Germany). For doing so, a sample is centrifuged at room temperature (23 °C) for 1 min at 4000 rpm for complete phase separation. The phases are separated by pipetting and 5 g of each phase is transferred into a single usage cuvette having a diameter of 32 mm from SPECTRO Analytical Instruments GmbH (Kleve, Germany). The concentration is determined via X-ray fluorescence analysis using a SPECTRO XEPOS spectrometer, type 16004851 from SPECTRO Analytical Instruments GmbH (Kleve, Germany). The sample chamber is inertized with helium. The amount of the transition metal is determined using a method calibrated with the pure substances, for instance sodium tungstate and phosphoric acid (75%).

The epoxide in the biphasic mixture obtained from an epoxidation process is preferably formed in an oxidation of an ethylenically unsaturated organic compound as defined above, wherein the peroxide more preferably is monoepoxycyclododecane. The amount of epoxide can for example be determined by gas chromatography with a flame ionization detector (GC-FID) using, for instance, a GC-2014 gas chromatograph from Shimadzu Deutschland GmbH (Duisburg, Germany). The determination can be carried out as follows: a sample of the biphasic mixture is centrifuged at room temperature (23 °C) for 1 min at 4000 rpm for obtaining a complete phase separation. 100 mg of the organic phase is weighed into a GC vial and diluted with standard solution by a factor of 10. The standard solution comprises 1 wt.-% of tetradecane and is further filled up with acetone. The measurement is performed using a Shimadzu GC-2014 gas chromatograph with a Supelcowax-10 column (length of 60 m, diameter of 0.32 mm and film thickness of 0.25 µm) with a flame ionization detector and a SPL-10 Split Injector. The sample is injected at 150 °C with a split of 1:10. The initial column temperature is 180 °C, which is hold for 10 min, then increased with a temperature ramp of 5 K/min to 200 °C and kept at this temperature for 35 min. Quantification of the products is determined by comparing the area of the products with the area of tetradecane as internal standard in correlation with the initial weight and a response factor, which is determined beforehand by recovery of the pure substances in a concentration series.

The quaternary ammonium compound in the biphasic mixture obtained from an epoxidation process preferably is the quaternary ammonium compound as defined above, more preferably the quaternary ammonium compound is methyltrioctylammonium or Adogen^{®} 464. The amount of the quaternary ammonium compound can be determined using 1H-NMR spectroscopy measured for instance by using a 500 MHz NMR spectrometer from Bruker Corporation (Massachusetts, USA). The determination can be carried out as follows: 20 mg of the organic sample is diluted twenty-fold with a mixture of DMSO-d6 and CDCl₃ (V/V = 1/1) and 8 mg 1,2,4,5-tetrachloro-3-nitrobenzene (TCNB) are added as an internal standard. The sample is measured with a 500 MHz Bruker NMR- spectrometer with a Bruker Avance III HD Console and the chromatogram is analyzed using the software TopSpin 3.6. Quantification of the quaternary ammonium compound is determined by the ratio of the 1H-proton signals of TCNB and a methyl signal of the ammonium compound.

Using these absolute methods as references the concentration of epoxide and ethylenically unsaturated organic compound can also be quantified online during the reaction using a Kaiser Optical Systems Rxn2 Raman analyzer from Endress+Hauser Group Services AG (Reinach, Switzerland) with a 785 nm laser, and immersion short focus probes and indirect hard modeling to quantify CDEN and CDAN-epoxide concentration and a partial least-square model for tungstate and hydrogen peroxide employing the Peaxact Software from S-PACT (Aachen, Germany). An online measurement as used herein refers to a method taking place continuously but not, as for inline measurements, directly in the process but, for example, in a bypass, through which the reactor content is continuously passed.

The biphasic mixture obtained from an epoxidation process as described above can be used again in an epoxidation process by adding the biphasic mixture to the reaction mixture of an epoxidation process. In particular, the biphasic mixture can be obtained from a continuous epoxidation process, for example the process described herein above, and can be used later in the same continuous process. The biphasic mixture can preferably be obtained from the epoxidation according to the method described herein above using the method for reactivating a homogeneous catalyst system as described herein above.

### Figures

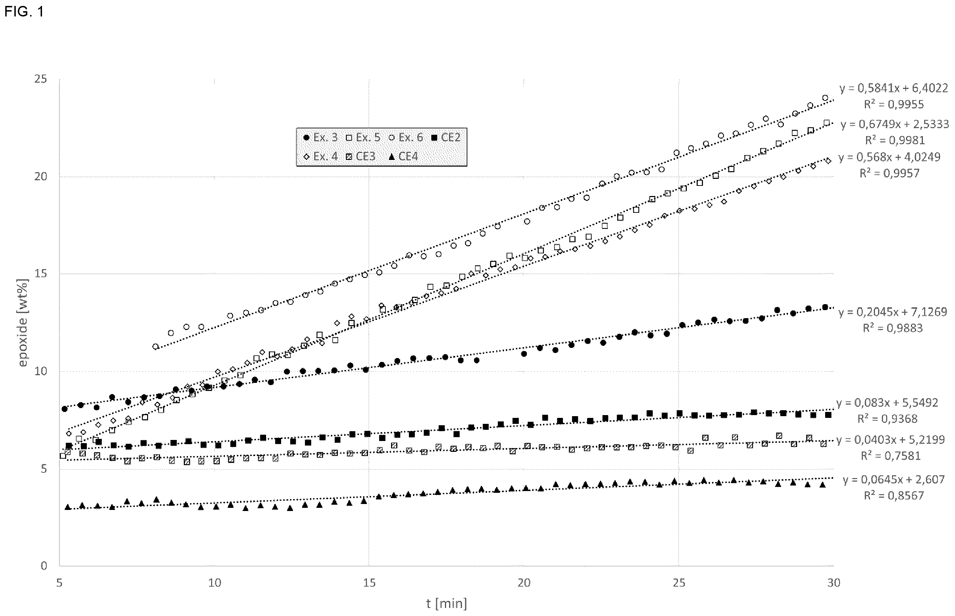

Figure 1 shows the progress of the epoxidation of CDEN with hydrogen peroxide using catalysts reactivated as described in Examples 3 to 6, including a treatment with an acid in the presence of hydrogen peroxide before combining the catalyst again with CDEN, and Comparative Examples 2 to 4, including a treatment with an acid but no hydrogen peroxide before combining the catalyst again with CDEN.

### Examples

### Example 1

An epoxidation of cyclic unsaturated C12 compounds was carried out in a continuous process in a cascade of three stirred tank reactors. The cascade comprised two reactors each having a 5 liter nominal capacity and, as a final stage, a third stirred tank reactor having a 25 liter nominal capacity. The content of the first two reactors was heated in an oil bath to 90 °C and that of the final reactor was heated in an oil bath to 80 °C.

To the first reactor 1.5 kg/h of cyclic unsaturated C12 compound (94 wt.-% CDEN and 6 wt.-% of CDAN), Adogen^{®} 464 methyl sulfate (PTC), sodium tungstate, phosphoric acid, sulfuric acid and a 60% H₂O₂ solution was fed. The pH value of the reaction mixture was adjusted to a pH of 1.60 by adding sulfuric acid.

The reaction mixture was passed into a second reactor. In addition, a further quantity of H₂O₂ was metered into the second reactor. In total, a ratio of 1.01 to 1.06 mol H₂O₂ per mol of CDEN was added to the first and second reactor.

The biphasic reaction mixture was passed from the second reactor into the third reactor and from there into a phase separation vessel to let the organic phase separate from the aqueous phase. The organic phase was supplied to a continuous membrane system using a pump and the aqueous phase was discarded. The residence time in each of the first and second reactor was approximately 2.5 h, while in the third reactor it was approximately 12.5 h. Residence time, as used herein, is the total average amount of time a discrete quantity of reagent spends inside the reactor. For an ideal continuously stirred-tank reactor, the theoretical residence time is equal to the reactor volume divided by the fluid flow rate.

The organic phase was fed to a membrane unit employing a oNF-2^{®} from Borsig Membrane Technology GmbH (Gladbeck, Germany) operating at 60 °C and a transmembrane-pressure of 40 bar. The organic phase was separated into permeate and retentate in a way such that 10 wt% of the feed was obtained as retentate and 90 wt% as permeate. The membrane was used as 2,5"x20" spiral-wound element to provide a sufficient permeate flow to process the feedflow of the organic phase and to provide an excess permeate-flow at the same time. The excess permeate-flow not needed for the permeate access was recycled to the feed.

The retentate was fed into a further stirred tank with a nominal capacity of 5 liter.

A 1.0 M aqueous sodium hydroxide solution was added and the biphasic mixture was adjusted to a pH of 8.5. The pH value was monitored using an online pH electrode and controlled with manual measurements using a Knick MEMO SES SE55X/1-NMSN sensor and a Knick Portavo 940X Multi 84461/205892 pH meter both from Knick Elektronische Messgeräte GmbH & Co. KH (Berlin, Germany).

The biphasic mixture having a pH of 8.5 was transferred into a stirred vessel with a nominal capacity of 5 liter referred to as hydrolysis reactor. Subsequently, the reaction mixture was recycled into the first reactor of the cascade.

The concentrations of CDEN, CDAN-epoxide and tungstate were monitored online employing Raman as described above and offline samples were taken every 24 hours until the reaction reaches a steady state and concentrations did not change anymore.

To accelerate the process of reaching a steady state, all reactors were initially filled with an organic and an aqueous phase with a tungstate concentration close to the expected steady state concentration, as shown in Table 1 below.

**Table 1: Amount of tungsten in the organic and aqueous phases in the reactors.**

| | Epoxidation Reactor 1 | Epoxidation Reactor 2 | Epoxidation Reactor 3 | Hydrolysis Reactor | Buffer Vessel |
|---|---|---|---|---|---|
| W [ppm] in org. phase | 1500 | 1500 | 2250 | 2500 | 2500 |
| W [ppm] in aq. phase | 3500 | 3500 | 50 | 50000 | 50000 |

When the concentrations of CDEN, CDAN-epoxide and tungstate did not change anymore, i.e., the steady state was reached, samples were taken every 12 hours while continuing with the online measurements as described above.

The activity of the first reactor was determined as described below.

### Example 2

In Example 2 an epoxidation of cyclic unsaturated C12 compounds was carried out using the same conditions as described in Example 1, except that a 1.0 M aqueous sodium hydroxide solution was added to adjust the pH of the biphasic mixture to a pH of 11.3. The activity in the first reactor after addition of the reactivated catalyst system was determined as described below.

### Comparative Example 1

In Comparative Example 1 an epoxidation of cyclic unsaturated C12 compounds was carried out using the same conditions as described in Example 1, except that hydrogen peroxide (in total a ratio of 1.01 to 1.06 mol H₂O₂ per mol of CDEN) was dosed in all three of the cascade of stirred tank reactors, the pH of the biphasic mixture was adjusted to a pH of 11.0, and the biphasic mixture was acidified in a 5 liter stirred tank reactor before being recycled into the first reactor of the cascade. More specifically, after adjusting the pH to a basic pH of 11.0, the resulting biphasic mixture was subsequently added into a further stirred tank and treated with sulfuric acid until a pH of 2 was achieved. The resulting mixture having a pH of 2 was recycled into the first reactor of the cascade.

When the concentrations of CDEN and CDAN-epoxide did not change anymore, samples were taken every 12 hours while continuing with the online measurements. Contrary to Example 1 and 2 the tungstate concentration is not constant but continuously increases without additional increase of catalyst activity.

After the resulting reaction mixtures of Example 1 and 2 and Comparative Example 1 were recycled into the first reactor of each cascade, the activity in the first reactors was determined as follows:
At constant pH, constant temperature, constant catalyst concentration and hydrogen peroxide concentration above the saturation level the activity of the catalyst in a first example "A" was compared to the activity of the catalyst in a second example "B" by dividing the reaction rate derived in example "A" by the reaction rate in example "B" using the following formula. $relative activy difference = \frac{\frac{c CDEN Reactor in \left(example A\right)}{c CDEN Reactor out \left(example A\right)} - 1}{\frac{c CDEN Reactor in \left(example B\right)}{c CDEN Reactor out \left(example B\right)} - 1}$ with *"c CDEN Reactor in"* being the total concentration of CDEN in the organic phase that was fed to the first reactor in form of the fresh CDEN and contained the biphasic mixture fed into the reactor in step (iva) and (ivb) of the present method and *"c CDEN Reactor out"* being the concentration of CDEN in the first reactor at steady state. Example A and example B relate to the present examples and comparative example depending on which activities of which examples are to be compared.

The amount (wt.-%) of CDEN in the first reactor after addition of the reactivated catalyst system was determined according to the Raman and GC measurements as described in detail above.

The amount of tungsten in the organic phase has been kept constant at an amount of 2500 ppm determined according to XRF and Raman measurements as described in detail above.

**Table 2: Activity of the first reactor in a continuous epoxidation process**

| | **Example 1** | **Example 2** | **Comparative Example 1** |
|---|---|---|---|
| CDEN (wt.-%) in first reactor | 9.4 | 12.3 | 28.1 |
| CDEN (wt.-%) in second reactor | 1.4 | 2.5 | 14.0 |
| CDEN (wt.-%) in third reactor | 1.4 | 2.5 | 4 |
| **Relative activity of first reactor in comparison to CE1** | 3.5 | 2.6 | 1 |
| CDEN (mol) per mol tungstate | 11458 | 11458 | 1432 |
| CDEN (mol) per mol PTC | 2615 | 2615 | 2091 |
| CDEN (mol) per mol phosphate | 1062 | 1062 | 531 |
| W (ppm) in org. phase | 2250 | 2250 | not constant, up to 3500 ppm |

As can be seen from the table above, it has surprisingly been found that when reactivating the homogeneous catalyst system according to the method of the present invention (Examples 1 and 2), the reactivity in the first reactor in a continuous oxidation process was significantly improved, compared to a reaction procedure in which the recycled biphasic mixture is acidified before being recycled into the first reactor (Comparative Example 1).

This is particularly surprising, as contacting hydrogen peroxide with a base should normally be avoided to prevent its decomposition. Nevertheless, it was possible to achieve significantly better efficiency with the method according to the present invention.

It is also surprising in light of the fact that in a study published by Rhodes at al. (Can. J. Chem. 2003, 81, 1044) it had been shown that complete decomposition of a tungsten heteropolyoxometalate only occurs at a pH value of 12. This might suggest that a pH of equal to or greater than 12 would be necessary to reactivate the transition metal from an inactive heteropolyoxometalate form. However, it has been shown in the present invention that adjusting the pH of the biphasic mixture comprising a permanently ionized phase transfer reagent to 8.5 (in step (iii)) a significantly increased activity of the reactivated catalyst can be achieved.

Accordingly, a higher amount of ethylenically unsaturated organic compound (CDEN) can be oxidized with the same amount of catalyst system. Thus, addition of fresh catalyst in a continuous process can be minimized, which reduces both costs and environmental impact.

### Examples 3 to 6 and Comparative Examples 2 to 4

Aliquots of 10 g of the biphasic mixture having a basic pH from the continuous process described in Example 1 were collected.

In Example 3, the biphasic mixture was acidified to about pH 1.6 using 2.5 N sulfuric acid. Hydrogen peroxide was added then immediately thereafter to treat the mixture with an acid in the presence of hydrogen peroxide.

In Examples 4 to 6 and Comparative Examples 2 to 4, the phases of the aliquots were separated and the aqueous phase (about 4.5 g) was acidified to about pH 1.6 using 2.5 N sulfuric acid to reactivate the catalyst in the presence or absence of hydrogen peroxide as described in the following.

To test the catalytic activity of the reactivated catalysts, the reactivated catalysts obtained in each of these (comparative) examples were then used in batch experiments as the only catalysts source (no fresh transition metal species, phase transfer reagent or phosphoric acid added) to oxidize CDEN using an excess of hydrogen peroxide at 90 °C. The progress of the oxidation reaction (formation of epoxide) over time was monitored using Raman spectroscopy as described above. The results are shown in Figure 1.

In Example 3, immediately after adding the sulfuric acid to the biphasic mixture, hydrogen peroxide (3 g) was added. The mixture obtained was stirred for 1 h at room temperature.

To test the catalytic activity of the reactivated catalyst, 95 g of CDEN and 10 g of water were heated in a reactor to a temperature of 95 °C.

The treated biphasic mixture was added to the reactor to start the epoxidation reaction. Additional portions of hydrogen peroxide (total amount 18 g) were added 15, 30 and 45 min after the start of the reaction to ensure that an excess of hydrogen peroxide was always present.

In Example 4, immediately after adding the sulfuric acid to the aqueous phase, hydrogen peroxide (3 g) was added. The mixture obtained was stirred for 1 h at room temperature, before it was filtered to remove any remaining solids.

To test the catalytic activity of the reactivated catalyst, 95 g of CDEN were heated in a reactor to a temperature of 95 °C. 10 g of water and 2.5 N sulfuric acid were added to the organic phase separated from the biphasic mixture to adjust the pH of this mixture to about 1.6.

Both, the treated aqueous phase and the acidified organic phase were added to the reactor to start the epoxidation reaction. Additional portions of hydrogen peroxide (total amount 18 g) were added 15, 30 and 45 min after the start of the reaction to ensure that an excess of hydrogen peroxide is always present.

Example 5 was carried out as described for Example 4, except that the mixture obtained from adding the sulfuric acid and hydrogen peroxide to the aqueous phase was stirred overnight, before it was filtered to remove any remaining solids.

Example 6 was carried out as described for Example 5, except that the mixture obtained from adding the sulfuric acid and hydrogen peroxide to the aqueous phase was not filtered after having been stirred overnight.

Comparative Example 2 was carried out as described for Example 4, except that the mixture obtained from adding the sulfuric acid was filtered before hydrogen peroxide was added. The resulting mixture was stirred for 1 h.

Comparative Example 3 was carried out as described for Example 5, except that no hydrogen peroxide was added after adding the sulfuric acid to the aqueous phase. Rather, the respective amount of peroxide was additionally added into the reactor at the start of the epoxidation reaction.

Comparative Example 4 was carried out as described for Example 6, except that no hydrogen peroxide was added after adding the sulfuric acid to the aqueous phase. Rather, the respective amount of peroxide was additionally added into the reactor at the start of the epoxidation reaction.

It can be seen from Figure 1 that an acidic treatment of the catalyst in the absence of peroxide (Comparative Examples 2 to 4) gives a significantly lower activity of the catalyst, as indicated by a lower slope of the regression line, in a following epoxidation reaction in comparison to an acidic treatment of the catalyst in the presence of peroxide (Examples 3 to 6). It further can be seen that removing solids formed or remaining in the treatment step, for instance by filtration, may enhance the catalyst's activity (the regression line of Example 5 having a larger slope than that of Example 6), while removing solids before the treating the mixture or the aqueous phase thereof lowers the catalyst's activity (Comparative Example 2 vs. Example 3).

## Claims

1. A method for reactivating a homogeneous catalyst system for the oxidation of an ethylenically unsaturated organic compound with a peroxide as oxidant in a reaction mixture comprising an organic phase, an acidic aqueous medium and a permanently ionized phase transfer reagent, comprising the steps of
(i) separating the organic phase comprising at least a part of the catalyst system from the aqueous medium;
(ii) concentrating the catalyst system in the organic phase;
(iii) adding at least one aqueous base to the organic phase comprising the catalyst system obtained in step (ii) to form a biphasic mixture having a pH of at least 7.0, determined in the stirred biphasic mixture using a pH electrode; and
(iva) feeding the biphasic mixture obtained in step (iii) to a reaction mixture comprising an ethylenically unsaturated organic compound, a peroxide and an acidic aqueous medium without separating the aqueous phase from the organic phase and without acidifying said mixture before feeding it to the reaction mixture; or
(ivb) treating either the biphasic mixture obtained in step (iii) or, after phase separation, the aqueous phase thereof, with an acid in the presence of a peroxide before feeding the treated biphasic mixture or both, the treated aqueous phase and the organic phase obtained from separating the biphasic mixture, respectively, to a reaction mixture comprising an ethylenically unsaturated organic compound and an acidic aqueous medium;
wherein the catalyst system comprises at least one derivative of a transition metal of Group IVb, Vb and Vlb in its highest oxidation state.

2. The method according to claim 1, wherein the permanently ionized phase transfer reagent comprises a quaternary ammonium compound, preferably an ammonium cation of the general formula N⁺R¹R²R³R⁴, wherein R¹, R², R³, and R⁴ are alkyl groups which each independently may be the same or different, wherein the ammonium cation has in total more than 10, preferably more than 15 carbon atoms in the alkyl groups, and wherein more preferably at least one of R¹, R², R³, and R⁴ is a methyl group.

3. The method according to any of the preceding claims, wherein the at least one derivative of the transition metal of Group IVb, Vb and Vlb is selected from a derivative of tungsten, molybdenum and vanadium, wherein the derivative preferably is selected from salts of H₂WO₄ or H₂MoO₄, or homo- or heteropolyoxometalates formed therefrom, more preferably from alkali or alkaline earth metal salts of H₂WO₄ or H₂MoO₄, or homo- or heteropolyoxometalates formed therefrom, and most preferably is Na₂WO₄ or a homo- or heteropolyoxometalate formed therefrom.

4. The method according to any of the preceding claims, wherein the oxidant is hydrogen peroxide.

5. The method according to any of the preceding claims, wherein step (ii) comprises separating an oxidized organic compound from the organic phase comprising the catalyst system by membrane filtration to concentrate the catalyst in the remaining organic phase.

6. The method according to any of the preceding claims, wherein in step (iii) the biphasic mixture has a pH of from 7.0 to 11.0, preferably of from 8.0 to 10.0, more preferably of from 8.0 to 9.0.

7. The method according to any of the preceding claims, wherein the reactivation is carried out in a continuous process, wherein the continuous process is carried out in a stirred tank reactor or in a series of stirred tank reactors.

8. A method for the oxidation of an ethylenically unsaturated organic compound, comprising the steps of
(i) oxidizing an ethylenically unsaturated organic compound with a peroxide in a reaction mixture comprising a homogeneous catalyst system, an organic phase, an acidic aqueous medium and a permanently ionized phase transfer reagent; wherein the homogeneous catalyst system comprises at least one derivative of a transition metal of Group IVb, Vb and Vlb in its highest oxidation state; and
(ii) reactivating the catalyst system of the reaction mixture according to the method of any of claims 1 to 7.

9. The method according to claim 8, wherein the oxidation comprises an epoxidation of the ethylenically unsaturated organic compound, wherein the ethylenically unsaturated organic compound preferably has a solubility in water at 20 °C of no more than 1 wt.-%, preferably of no more than 0.5 wt.-%.

10. The method according to claim 8 or claim 9, wherein the oxidation is carried out at a pH of 4 or less, preferably at a pH of 1.5 to 4.0.

11. The method according to any of claims 8 to 10, wherein the catalyst system further comprises phosphoric acid and/or a salt thereof.

12. A method for synthesizing a lactam, comprising:
epoxidizing a cyclic ethylenically unsaturated compound to an epoxide;
rearranging the epoxide to a ketone;
converting the ketone to an oxime; and
rearranging the oxime to the lactam;
wherein the cyclic ethylenically unsaturated compound is epoxidized by at least one peroxide in a reaction mixture comprising an organic phase, an acidic aqueous medium, a permanently ionized phase transfer reagent and a catalyst system comprising at least one derivative of a transition metal of Group IVb, Vb and Vlb in its highest oxidation state, and
wherein the catalyst system is reactivated according to the method of any of claims 1 to 7.

13. A biphasic mixture for use in an oxidation of an ethylenically unsaturated organic compound, comprising an oxidation product of the ethylenically unsaturated organic compound, an aqueous phase and a catalyst system as defined in any of claims 1 to 3, wherein the biphasic mixture has a pH value of at least 7.0, determined in the stirred biphasic mixture using a pH electrode, preferably of from 7.0 to 11.0, more preferably of from 8.0 to 10.0, and most preferably of from 8.0 to 9.0.

14. A biphasic mixture according to claim 13, wherein the biphasic mixture is obtained from an epoxidation process comprising an organic phase and an aqueous phase, wherein the organic phase comprises an epoxide in an amount of at least 50 wt.-%, preferably of at least 70 wt.-%, more preferably of at least 80 wt.-%, a transition metal of the Group IVb, Vb and Vlb in an amount of less than 6000 ppm, preferably less than 1500 ppm, and a quaternary ammonium compound in an amount of more than 0.2 wt.-%, preferably more than 0.5 wt.-%, more preferably more than 1.0 wt.-%, wherein ppm and wt.-% are based on the total amount of the organic phase, and wherein the aqueous phase comprises the same transition metal as the organic phase in such an amount that more than 50 wt.-% of the total amount of said transition metal in the biphasic mixture is in the aqueous phase, preferably more than 75 wt.-%, more preferably more than 85 wt.-%.

15. The biphasic mixture according to claim 14, wherein the transition metal is tungsten, molybdenum or vanadium, preferably tungsten; and/or wherein the epoxide is formed in an oxidation of an ethylenically unsaturated organic compound preferably having a solubility in water at 20 °C of no more than 1 wt.-%, preferably of no more than 0.5 wt.-%; and/or wherein the quaternary ammonium compound is an ammonium cation of the general formula N⁺R¹R²R³R⁴, wherein R¹, R², R³, and R⁴ are alkyl groups which each independently may be the same or different, wherein the ammonium cation has in total more than 10, preferably more than 15 carbon atoms in the alkyl groups, and wherein more preferably at least one of R', R², R³, and R⁴ is a methyl group.

## Patentansprüche

1. Verfahren zur Reaktivierung eines homogenen Katalysatorsystems für die Oxidation einer ethylenisch ungesättigten organischen Verbindung mit einem Peroxid als Oxidationsmittel in einem Reaktionsgemisch, das eine organische Phase, ein saures wässriges Medium und ein permanent ionisiertes Phasentransferreagenz umfasst, umfassend die Schritte
(i) Abtrennen der organischen Phase, die mindestens einen Teil des Katalysatorsystems umfasst, von dem wässrigen Medium;
(ii) Konzentrieren des Katalysatorsystems in der organischen Phase;
(iii) Zugeben mindestens einer wässrigen Base zu der organischen Phase, die das in Schritt (ii) erhaltene Katalysatorsystem umfasst, um ein zweiphasiges Gemisch mit einem pH-Wert von mindestens 7,0 zu bilden, bestimmt in dem gerührten zweiphasigen Gemisch unter Verwendung einer pH-Elektrode; und
(iva) Zuführen des in Schritt (iii) erhaltenen zweiphasigen Gemischs zu einer Reaktionsmischung, die eine ethylenisch ungesättigte organische Verbindung, ein Peroxid und ein saures wässriges Medium umfasst, ohne die wässrige Phase von der organischen Phase zu trennen und ohne das Gemisch anzusäuern, bevor es der Reaktionsmischung zugeführt wird; oder
(ivb) Behandeln entweder des in Schritt (iii) erhaltenen zweiphasigen Gemischs oder, nach Phasentrennung, der wässrigen Phase davon mit einer Säure in Gegenwart eines Peroxids, bevor das behandelte zweiphasige Gemisch oder beide, die behandelte wässrige Phase bzw. die organische Phase, die aus der Trennung des zweiphasigen Gemischs erhalten wurde, einer Reaktionsmischung, die eine ethylenisch ungesättigte organische Verbindung und ein saures wässriges Medium umfasst, zugeführt werden;
wobei das Katalysatorsystem mindestens ein Derivat eines Übergangsmetalls der Gruppe IVb, Vb und VIb in seiner höchsten Oxidationsstufe umfasst.

2. Verfahren nach Anspruch 1, wobei das permanent ionisierte Phasentransferreagenz eine quartäre Ammoniumverbindung, vorzugsweise ein Ammoniumkation der allgemeinen Formel N⁺R¹R²R³R⁴, worin R¹, R², R³ und R⁴ Alkylgruppen sind, die jeweils unabhängig gleich oder verschieden sein können, umfasst, wobei das Ammoniumkation insgesamt mehr als 10, vorzugsweise mehr als 15 Kohlenstoffatome in den Alkylgruppen aufweist, und wobei besonders bevorzugt mindestens eines von R¹, R², R³ und R⁴ eine Methylgruppe ist.

3. Verfahren nach einem der vorhergehenden Ansprüche, wobei das mindestens eine Derivat des Übergangsmetalls der Gruppe IVb, Vb und VIb ausgewählt ist aus einem Derivat von Wolfram, Molybdän und Vanadium, wobei das Derivat bevorzugt ausgewählt ist aus Salzen von H₂WO₄ oder H₂MoO₄ oder daraus gebildeten Homo- oder Heteropolyoxometallaten, besonders bevorzugt aus Alkali- oder Erdalkalimetallsalzen von H₂WO₄ oder H₂MoO₄ oder daraus gebildeten Homo- oder Heteroometallaten, und ganz besonders bevorzugt Na₂WO₄ oder ein daraus gebildetes Homo- oder Heteropolyoxometallat ist.

4. Verfahren nach einem der vorhergehenden Ansprüche, wobei das Oxidationsmittel Wasserstoffperoxid ist.

5. Verfahren nach einem der vorhergehenden Ansprüche, wobei Schritt (ii) das Abtrennen einer oxidierten organischen Verbindung von der organischen Phase, die das Katalysatorsystem umfasst, durch Membranfiltration umfasst, um den Katalysator in der verbleibenden organischen Phase zu konzentrieren.

6. Verfahren nach einem der vorhergehenden Ansprüche, wobei in Schritt (iii) das zweiphasige Gemisch einen pH-Wert von 7,0 bis 11,0, bevorzugt von 8,0 bis 10,0, besonders bevorzugt von 8,0 bis 9,0 aufweist.

7. Verfahren nach einem der vorhergehenden Ansprüche, wobei die Reaktivierung in einem kontinuierlichen Verfahren durchgeführt wird, wobei das kontinuierliche Verfahren in einem Rührkesselreaktor oder in einer Reihe von Rührkesselreaktoren durchgeführt wird.

8. Verfahren zur Oxidation einer ethylenisch ungesättigten organischen Verbindung, umfassend die Schritte
(i) Oxidieren einer ethylenisch ungesättigten organischen Verbindung mit einem Peroxid in einer Reaktionsmischung, die ein homogenes Katalysatorsystem, eine organische Phase, ein saures wässriges Medium und ein permanent ionisiertes Phasentransferreagenz umfasst; wobei das homogene Katalysatorsystem mindestens ein Derivat eines Übergangsmetalls der Gruppe IVb, Vb und VIb in seiner höchsten Oxidationsstufe umfasst; und
(ii) Reaktivierung des Katalysatorsystems der Reaktionsmischung gemäß dem Verfahren nach einem der Ansprüche 1 bis 7.

9. Verfahren nach Anspruch 8, wobei die Oxidation eine Epoxidierung der ethylenisch ungesättigten organischen Verbindung umfasst, wobei die ethylenisch ungesättigte organische Verbindung vorzugsweise eine Löslichkeit in Wasser bei 20 °C von nicht mehr als 1 Gew.-%, vorzugsweise von nicht mehr als 0,5 Gew.-% aufweist.

10. Verfahren nach Anspruch 8 oder Anspruch 9, wobei die Oxidation bei einem pH-Wert von 4 oder weniger, vorzugsweise bei einem pH-Wert von 1,5 bis 4,0, durchgeführt wird.

11. Verfahren nach einem der Ansprüche 8 bis 10, wobei das Katalysatorsystem ferner Phosphorsäure und/oder ein Salz davon umfasst.

12. Verfahren zum Synthetisieren eines Lactams, umfassend:
Epoxidieren einer cyclischen ethylenisch ungesättigten Verbindung zu einem Epoxid;
Umordnen des Epoxids zu einem Keton;
Umwandeln des Ketons zu einem Oxim; und
Umordnen des Oxims zu dem Lactam;
wobei die cyclische ethylenisch ungesättigte Verbindung durch mindestens ein Peroxid in einer Reaktionsmischung epoxidiert wird, die eine organische Phase, ein saures wässriges Medium, ein permanent ionisiertes Phasentransferreagenz und ein Katalysatorsystem umfasst, das mindestens ein Derivat eines Übergangsmetalls der Gruppe IVb, Vb und VIb in seiner höchsten Oxidationsstufe umfasst, und
wobei das Katalysatorsystem gemäß dem Verfahren nach einem der Ansprüche 1 bis 7 reaktiviert wird.

13. Zweiphasiges Gemisch zur Verwendung bei einer Oxidation einer ethylenisch ungesättigten organischen Verbindung, umfassend ein Oxidationsprodukt der ethylenisch ungesättigten organischen Verbindung, eine wässrige Phase und ein Katalysatorsystem wie in einem der Ansprüche 1 bis 3 definiert, wobei das zweiphasige Gemisch einen pH-Wert von mindestens 7,0, bestimmt in dem gerührten zweiphasigen Gemisch unter Verwendung einer pH-Elektrode, bevorzugt von 7,0 bis 11,0, besonders bevorzugt von 8,0 bis 10,0, und ganz besonders bevorzugt von 8,0 bis 9,0 aufweist.

14. Zweiphasiges Gemisch nach Anspruch 13, wobei das zweiphasige Gemisch aus einem Epoxidierungsprozess erhalten wird, der eine organische Phase und eine wässrige Phase umfasst, wobei die organische Phase ein Epoxid in einer Menge von mindestens 50 Gew.-%, bevorzugt von mindestens 70 Gew.-%, besonders bevorzugt von mindestens 80 Gew.-%, ein Übergangsmetall der Gruppe IVb, Vb und VIb in einer Menge von weniger als 6000 ppm, bevorzugt von weniger als 1500 ppm, und eine quartäre Ammoniumverbindung in einer Menge von mehr als 0,2 Gew.-%, bevorzugt mehr als 0,5 Gew.-%, besonders bevorzugt mehr als 1,0 Gew.-%, wobei ppm und Gew.-% auf die Gesamtmenge der organischen Phase bezogen sind, umfasst, und wobei die wässrige Phase das gleiche Übergangsmetall wie die organische Phase in einer solchen Menge umfasst, dass mehr als 50 Gew.-% der Gesamtmenge des Übergangsmetalls in dem zweiphasigen Gemisch in der wässrigen Phase vorliegen, bevorzugt mehr als 75 Gew.-%, besonders bevorzugt mehr als 85 Gew.-%.

15. Zweiphasiges Gemisch nach Anspruch 14, wobei das Übergangsmetall Wolfram, Molybdän oder Vanadium, vorzugsweise Wolfram ist; und/oder wobei das Epoxid in einer Oxidation einer ethylenisch ungesättigten organischen Verbindung gebildet wird, die vorzugsweise eine Löslichkeit in Wasser bei 20 °C von nicht mehr als 1 Gew.-%, vorzugsweise von nicht mehr als 0,5 Gew.-% aufweist; und/oder wobei die quartäre Ammoniumverbindung ein Ammoniumkation der allgemeinen Formel N⁺R¹R²R³R⁴ ist, wobei R¹, R², R³ und R⁴ Alkylgruppen sind, die jeweils unabhängig gleich oder verschieden sein können, wobei das Ammoniumkation insgesamt mehr als 10, vorzugsweise mehr als 15 Kohlenstoffatome in den Alkylgruppen aufweist, und wobei weiter bevorzugt mindestens eines von R¹, R², R³ und R⁴ eine Methylgruppe ist.

## Revendications

1. Procédé de réactivation d'un système de catalyseur homogène pour l'oxydation d'un composé organique éthyléniquement insaturé avec un peroxyde en tant qu'oxydant dans un mélange réactionnel comprenant une phase organique, un milieu aqueux acide et un réactif de transfert de phases ionisé de façon permanente, comprenant les étapes de
(i) séparation de la phase organique comprenant au moins une partie du système de catalyseur du milieu aqueux ;
(ii) concentration du système de catalyseur dans la phase organique ;
(iii) ajout d'au moins une base aqueuse à la phase organique comprenant le système de catalyseur obtenu dans l'étape (ii) pour former un mélange biphasique ayant un pH d'au moins 7,0, déterminé dans le mélange biphasique agité à l'aide d'une électrode de pH ; et
(iva) alimentation du mélange biphasique obtenu à l'étape (iii) à un mélange réactionnel comprenant un composé organique éthyléniquement insaturé, un peroxyde et un milieu aqueux acide sans séparer la phase aqueuse de la phase organique et sans acidifier ledit mélange avant de l'alimenter dans le mélange réactionnel ; ou
(ivb) traitement soit du mélange biphasique obtenu à l'étape (iii), soit, après une séparation de phases, de sa phase aqueuse, par un acide en présence d'un peroxyde avant d'alimenter le mélange biphasique traité, soit les deux, la phase aqueuse traitée et la phase organique obtenues de la séparation du mélange biphasique respectivement, à un mélange réactionnel comprenant un composé organique éthyléniquement insaturé et un milieu aqueux acide ;
dans lequel le système de catalyseur comprend au moins un dérivé d'un métal de transition du groupe IVb, Vb et VIb dans son état d'oxydation le plus élevé.

2. Procédé selon la revendication 1, dans lequel le réactif de transfert de phases ionisé de façon permanente comprend un composé ammonium quaternaire, préférablement un cation ammonium de formule générale N⁺R¹R²R³R⁴, dans laquelle R¹, R², R³ et R⁴ sont des groupes alkyle qui peuvent chacun indépendamment être identiques ou différents, dans laquelle le cation ammonium a au total plus de 10, préférablement plus de 15 atomes de carbone dans les groupes alkyle, et dans laquelle plus préférablement au moins l'un parmi R¹, R², R³ et R⁴ est un groupe méthyle.

3. Procédé selon l'une quelconque des revendications précédentes, dans lequel l'au moins un dérivé du métal de transition du groupe IVb, Vb et VIb est choisi parmi un dérivé de tungstène, de molybdène et de vanadium, dans lequel le dérivé est de préférence choisi parmi des sels de H₂WO₄ ou H₂MoO₄, ou des homo- ou hétéropolyoxométalates formés à partir de ceux-ci, plus préférablement parmi des sels de métaux alcalins ou alcalino-terreux de H₂WO₄ ou H₂MoO₄, ou des homo- ou hétéropolyoxométalates formés à partir de ceux-ci, et le plus préférablement Na₂WO₄ ou un homo- ou hétéropolyoxométalate formé à partir de celui-ci.

4. Procédé selon l'une quelconque des revendications précédentes, dans lequel l'oxydant est le peroxyde d'hydrogène.

5. Procédé selon l'une quelconque des revendications précédentes, dans lequel l'étape (ii) comprend la séparation d'un composé organique oxydé de la phase organique comprenant le système de catalyseur par filtration sur membrane pour concentrer le catalyseur dans la phase organique restante.

6. Procédé selon l'une quelconque des revendications précédentes, dans lequel, dans l'étape (iii), le mélange biphasique a un pH de 7,0 à 11,0, de préférence de 8,0 à 10,0, plus préférablement de 8,0 à 9,0.

7. Procédé selon l'une quelconque des revendications précédentes, dans lequel la réactivation est effectuée dans un procédé continu, dans lequel le procédé continu est effectué dans un réacteur à cuve agitée ou dans une série de réacteurs à cuve agitée.

8. Procédé d'oxydation d'un composé organique éthyléniquement insaturé, comprenant les étapes de
(i) oxydation d'un composé organique éthyléniquement insaturé avec un peroxyde dans un mélange réactionnel comprenant un système de catalyseur homogène, une phase organique, un milieu aqueux acide et un réactif de transfert de phases ionisé de façon permanente ; le système de catalyseur homogène comprenant au moins un dérivé d'un métal de transition du groupe IVb, Vb et VIb dans son état d'oxydation le plus élevé ; et
(ii) réactivation du système de catalyseur du mélange réactionnel selon le procédé de l'une quelconque des revendications 1 à 7.

9. Procédé selon la revendication 8, dans lequel l'oxydation comprend une époxydation du composé organique éthyléniquement insaturé, dans lequel le composé organique éthyléniquement insaturé a de préférence une solubilité dans l'eau à 20 °C non supérieure à 1 % en poids, de préférence non supérieure à 0,5 % en poids.

10. Procédé selon la revendication 8 ou la revendication 9, dans lequel l'oxydation est effectuée à un pH de 4 ou moins, de préférence à un pH de 1,5 à 4,0.

11. Procédé selon l'une quelconque des revendications 8 à 10, dans lequel le système de catalyseur comprend en outre de l'acide phosphorique et/ou un sel de celui-ci.

12. Procédé de synthèse d'un lactame comprenant :
époxydation d'un composé éthyléniquement insaturé cyclique en un époxyde ;
réarrangement de l'époxyde en une cétone ;
conversion de la cétone en un oxime ; et
réarrangement de l'oxime en lactame ;
dans lequel le composé éthyléniquement insaturé cyclique est époxydé par au moins un peroxyde dans un mélange réactionnel comprenant une phase organique, un milieu aqueux acide, un réactif de transfert de phases ionisé de façon permanente et un système de catalyseur comprenant au moins un dérivé d'un métal de transition du groupe IVb, Vb et VIb dans son état d'oxydation le plus élevé, et
dans lequel le système de catalyseur est réactivé selon le procédé de l'une quelconque des revendications 1 à 7.

13. Mélange biphasique destiné à être utilisé dans une oxydation d'un composé organique éthyléniquement insaturé, comprenant un produit d'oxydation du composé organique éthyléniquement insaturé, une phase aqueuse et un système de catalyseur tel que défini dans l'une quelconque des revendications 1 à 3, le mélange biphasique ayant une valeur de pH d'au moins 7,0, déterminée dans le mélange biphasique agité en utilisant une électrode de pH, de préférence de 7,0 à 11,0, plus préférablement de 8,0 à 10,0, et le plus préférablement de 8,0 à 9,0.

14. Mélange biphasique selon la revendication 13, dans lequel le mélange biphasique est obtenu à partir d'un processus d'époxydation comprenant une phase organique et une phase aqueuse, dans lequel la phase organique comprend un époxyde en une quantité d'au moins 50 % en poids, de préférence d'au moins 70 % en poids, plus préférablement d'au moins 80 % en poids, un métal de transition du groupe IVb, Vb et VIb en une quantité inférieure à 6 000 ppm, de préférence inférieure à 1 500 ppm, et un composé ammonium quaternaire en une quantité supérieure à 0,2 % en poids, de préférence supérieure à 0,5 % en poids, plus préférablement supérieure à 1,0 % en poids, dans lequel les ppm et les % en poids sont basés sur la quantité totale de la phase organique, et dans lequel la phase aqueuse comprend le même métal de transition que la phase organique en une quantité telle que plus de 50 % en poids de la quantité totale dudit métal de transition dans le mélange biphasique se trouve dans la phase aqueuse, de préférence plus de 75 % en poids, plus préférablement plus de 85 % en poids.

15. Mélange biphasique selon la revendication 14, dans lequel le métal de transition est le tungstène, le molybdène ou le vanadium, de préférence le tungstène ; et/ou dans lequel l'époxyde est formé dans une oxydation d'un composé organique éthyléniquement insaturé ayant de préférence une solubilité dans l'eau à 20 °C non supérieure à 1 % en poids, de préférence non supérieure à 0,5 % en poids ; et/ou dans lequel le composé ammonium quaternaire est un cation ammonium de formule générale N⁺R¹R²R³R⁴, dans laquelle R¹, R², R³ et R⁴ sont des groupes alkyle qui peuvent chacun indépendamment être identiques ou différents, dans laquelle le cation ammonium a au total plus de 10, de préférence plus de 15 atomes de carbone dans les groupes alkyle, et dans laquelle plus préférablement au moins l'un parmi R¹, R², R³ et R⁴ est un groupe méthyle.
